(19) Europäisches Patentamt
European Patent Office
Office européen des brevets

(11) Publication number: **0 083 566 A1**

(12) **EUROPEAN PATENT APPLICATION**

(21) Application number: **83101610.0**

(22) Date of filing: **01.04.81**

(51) Int. Cl.³: **C 07 D 413/04**
// C07D261/08

(30) Priority: **10.04.80 US 138872**
**14.10.80 US 196785**
**10.04.80 US 138873**

(43) Date of publication of application: **13.07.83**
**Bulletin 83/28**

(84) Designated Contracting States: **AT BE CH DE FR GB IT LI LU NL SE**

(60) Publication number of the earlier application in accordance with Art. 76 EPC: **0038298**

(71) Applicant: **SANDOZ AG, Lichtstrasse 35, CH-4002 Basel (CH)**

(72) Inventor: **Brand, Leonard Jay, 114 Dover Chester Road, Randolph, N.J. 07801 (US)**
Inventor: **Nadelson, Jeffrey, 12 Benedict Crescent, Denville, N.J. 07834 (US)**

(54) **Isoxazolyl indoles.**

(57) Compounds of formula V

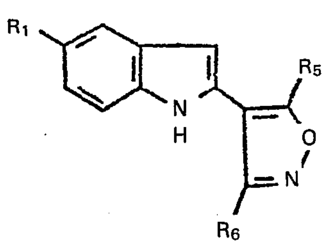

V

wherein
$R_1$ represents hydrogen, fluorine, chlorine, $C_{1-4}$alkyl or $C_{1-4}$alkoxy,
$R_5$ represents hydrogen or $C_{1-4}$alkyl and
$R_6$ represents hydrogen, $C_{1-4}$alkyl, phenyl or phenyl mono-substituted by fluorine, chlorine, $C_{1-4}$alkyl or $C_{1-4}$alkoxy.

These compounds may be used in the preparation of pharmacologically active substances.

EP 0 083 566 A1

ISOXAZOLYL INDOLES

The invention relates to compounds of formula V

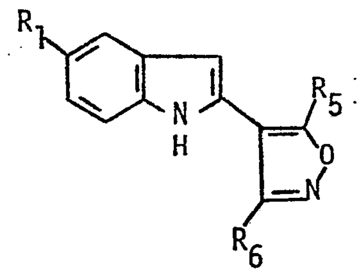

V

wherein $R_1$ represents hydrogen, fluorine, chlorine, $C_{1-4}$alkyl or $C_{1-4}$alkoxy,

$R_5$ represents hydrogen or $C_{1-4}$alkyl and

$R_6$ represents hydrogen, $C_{1-4}$alkyl, phenyl or phenyl mono-substituted by fluorine, chlorine, $C_{1-4}$alkyl or $C_{1-4}$alkoxy.

Compounds of formula V can be prepared by cyclising a compound of formula VII

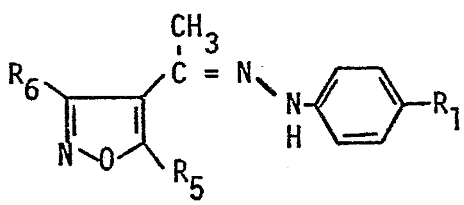

VII

wherein $R_1$, $R_5$ and $R_6$ are as defined above.

The cyclisation is preferably carried out in the presence of an acid such as acetic acid, p-toluenesulphonic acid or, preferably, polyphosphoric acid, and in the presence of an inert solvent such as an aromatic hydrocarbon e.g. benzene or toluene or preferably an excess of said acid. Preferred temperatures are of from about 70° to 150°C, most preferably 105-120°C.

Compounds of formula VII can be prepared by reacting a compound of formula VIII

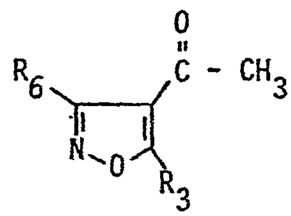

VIII

with a compound of formula IX

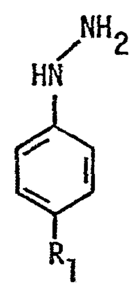

IX

wherein $R_1$, $R_5$ and $R_6$ are as defined above.

The reaction is preferably carried out in the presence of an acid such as sulphuric acid, a polyphosphoric acid or preferably p-toluene sulphonic acid and in the presence of an inert solvent. Examples of preferred solvents are aromatic hydrocarbons such as benzene or toluene or a lower alkanol such as methanol and most preferably ethanol. Preferred temperatures are of from about 0°-100°C, most preferably 20° to 35°C.

The products resulting from the above processes can be isolated and purified in conventional manner. Intermediate compounds can where appropriate be further reacted without isolation.

Insofar as the production of other starting materials is not described above, these are either known or can be prepared in conventional manner from known starting materials.

The compounds of formula V may be employed in the production of pharmacologically active end-products.

They may for example be reacted with a compound of formula VI

$$(COX)_2 \qquad VI$$

to give a compound of formula III

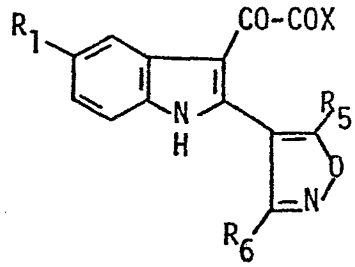

III

which in turn may be reacted with a compound of formula IV

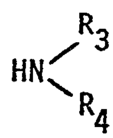

IV

to give a compound of formula II

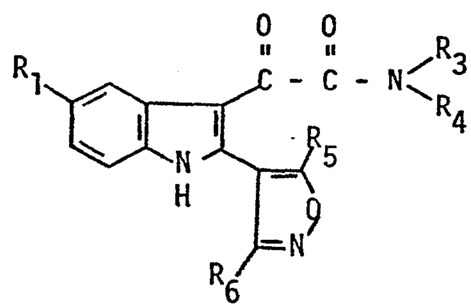

II

The compounds of formula II may in turn be reduced to give pharmacologically active compounds of formula I

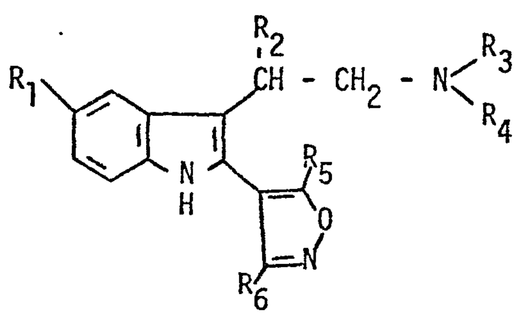

I

In the compounds of formulae I, II, III, IV and VI $R_1$, $R_5$ and $R_6$ are as defined for formula V, $R_2$ represents hydrogen or hydroxyl, $R_3$ and $R_4$ represent, independently, $C_{1-4}$alkyl or together with the adjacent nitrogen atom

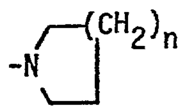 , 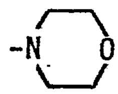 or 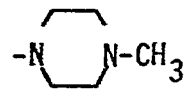

wherein n is 1, 2 or 3 and

X represents a leaving group such as chlorine or bromine.

The end-products of formula I are described and claimed in our co-pending application no. 81810131.3 (Publ. No. 0038298/A1) which also describes the processes outlined above and preferred substituents.

The following examples in which all temperatures are in °C and room temperature is 20-30°C, illustrate the invention.

EXAMPLE 1 : 1-(3-Ethyl-5-methyl-4-isoxazolyl)-1-ethanone phenyl hydrazone (compound of formula VII)

A mixture of 61.1 g (0.4 mol) of 4-acetyl-3-ethyl-5-methylisoxazole, 39.4 ml (0.4 mol) of phenyl hydrazine and 500 mg toluenesulphonic acid in 400 ml ethanol is stirred at room temperature for 48 hours. The resulting solid is filtered and washed with cold ether to give 1-(3-ethyl-5-methyl-4-isoxazolyl)-1-ethanone phenyl hydrazone; m.p. 72° to 75°.

EXAMPLE 2 : 2-(3-Ethyl-5-methyl-4-isoxazolyl)-indole (compound of formula V)

To 1350 grams of polyphosphoric acid at 100° to 110° there is added portionwise 74.5 g (0.307 mol) of 1-(3-ethyl-5-methyl-4-isoxazolyl)-1-ethanone phenyl hydrazone while maintaining the temperature between 105° and 115°. After addition is complete, the mixture is stirred at 100° to 110° for 3 hours. The mixture is then poured onto ice and water and the resulting gum extracted into methylene chloride. The methylene chloride is decolorized, dried over anhydrous magnesium sulphate, filtered and evaporated in vacuo to give 2-(3-ethyl-5-methyl-4-isoxazolyl)-indole.

The following compounds may be prepared analogously to Examples 1 and 2 or by any other method as hereinbefore described.

| Cmpd. No. | $R_4$ | $R_5$ | $R_6$ | III | V | VII | $R_3$ | $R_4$ | II |
|---|---|---|---|---|---|---|---|---|---|
| a | H | $CH_3$ | $C_2H_5$ | | | m.p. 72-75° | $CH_3$ | $CH_3$ | m.p. 221-223° |
| b | | H | $C_2H_5$ | | | | | | m.p. 211-212° |
| c | | $C_2H_5$ | $C_2H_5$ | | | | | | m.p. 218-219° |
| d | | $\underline{i}-C_3H_7$ | $C_2H_5$ | | | | | | |
| e | | $\underline{t}-C_4H_9$ | $C_2H_5$ | | | | | | |
| f | | $CH_3$ | $CH_3$ | | | | | | m.p. 211-214° |
| g | | $CH_3$ | H | | | | | | |
| h | | $CH_3$ | $\underline{n}-C_3H_7$ | | | | | | m.p. 153-154° |
| i | | $CH_3$ | $\underline{t}-C_4H_9$ | | | | | | m.p. 270° (dec.) |
| j | | $C_2H_5$ | $CH_3$ | | | | | | m.p. 155-156° |
| k | | $CH_3$ | $\underline{i}-C_3H_7$ | | | | | | m.p. 231-233° |
| l | F | | $C_2H_5$ | | | | | | |
| m | $CH_3$ | | | | | | | | |
| n | $OCH_3$ | | | | | | | | |
| o | H | | | | | | $C_2H_5$ | $C_2H_5$ | m.p. 2-1-213° |
| p | H | | | | | | 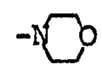 | | |
| q | H | | | | | | 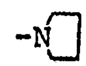 | | m.p. 194-197° |

| Cmpd. No. | $R_4$ | $R_5$ | $R_6$ | III | V | VII | $R_3$ | $R_4$ | II |
|---|---|---|---|---|---|---|---|---|---|
| r | H | $CH_3$ | $C_2H_5$ | | | | 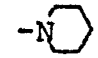 | | m.p. 199-203° |
| s | F | | $C_2H_5$ | | | | 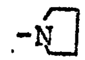 | | |
| t | H | | phenyl | | m.p.145-146° | m.p.129-133° | $CH_3$ | $CH_3$ | m.p. 259-261° |
| u | F | | | | | | $CH_3$ | $CH_3$ | |
| v | $CH_3$ | | | | | | $CH_3$ | $CH_3$ | |
| w | $OCH_3$ | | | | | | $CH_3$ | $CH_3$ | |
| x | H | | | | | | 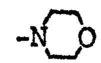 | | m.p. 210-221° |
| y | H | | | | | | 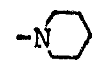 | | m.p. 193-195° |
| z | H | | | | | | 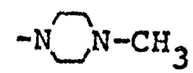 | | |
| aa | F | | $CH_3$ | | | | $CH_3$ | $CH_3$ | m.p. 220-222° |
| ab | F | $C_2H_5$ | $CH_3$ | | | | $CH_3$ | $CH_3$ | |
| ac | F | $CH_3$ | $CH_3$ | | | | 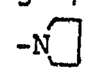 | | |
| ad | F | $CH_3$ | $CH_3$ | | | | 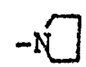 | | |
| ae | H | $CH_3$ | phenyl | | | | $C_2H_5$ | $C_2H_5$ | |

What is claimed is:

1. Compounds of formula V

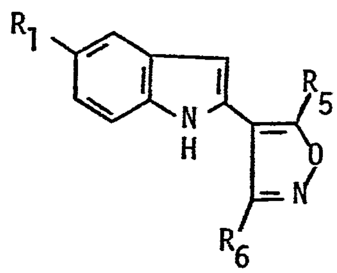

V

wherein $R_1$ represents hydrogen, fluorine, chlorine, $C_{1-4}$alkyl or $C_{1-4}$alkoxy,

$R_5$ represents hydrogen or $C_{1-4}$alkyl and

$R_6$ represents hydrogen, $C_{1-4}$alkyl, phenyl or phenyl monosubstituted by fluorine, chlorine, $C_{1-4}$alkyl or $C_{1-4}$alkoxy.

2. 2-(3-Ethyl-5-methyl-4-isoxazolyl)-indole.

3. A process for preparing a compound of formula V which comprises cyclising a compound of formula VII

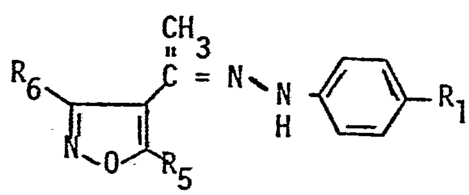

VII

wherein $R_1$, $R_5$ and $R_6$ are as defined above.

0083566

European Patent Office

# EUROPEAN SEARCH REPORT

Application number

EP 83 10 1610

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | US-A-3 467 670 (JOHN T. SUH)<br>--- | | C 07 D 413/04<br>C 07 D 261/08 |
| A | US-A-4 059 583 (DAVID FRED MCCOMSEY)<br>--- | | |
| A | GB-A-1 075 156 (INSTITUTO LUSO FARMACO D'ITALIA)<br>----- | | |
| | | | TECHNICAL FIELDS SEARCHED (Int. Cl. 3) |
| | | | C 07 D 413/00 |

The present search report has been drawn up for all claims

| Place of search | Date of completion of the search | Examiner |
|---|---|---|
| THE HAGUE | 14-04-1983 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82